Europäisches Patentamt

European Patent Office

Office européen des brevets

(19)

(11) Publication number: **0 015 123 B1**

## EUROPEAN PATENT SPECIFICATION

(12)

(45) Date of publication of patent specification: **22.12.82**

(51) Int. Cl.³: **C 12 P . 7/62, C 08 G 63/72**

(21) Application number: **80300431.6**

(22) Date of filing: **14.02.80**

(54) **A process for the extraction of poly-3-hydroxy-butyric acid from microbial cells.**

(30) Priority: **21.02.79 GB 7906076**
**21.02.79 GB 7906077**
**08.05.79 GB 7915858**

(43) Date of publication of application:
**03.09.80 Bulletin 80/18**

(45) Publication of the grant of the patent:
**22.12.82 Bulletin 82/51**

(84) Designated Contracting States:
**BE CH DE FR GB IT LU NL**

(56) References cited:
**US - A - 3 036 959**
**US - A - 3 044 942**

**MACROMOLECULES, vol. 9, no. 5, September-
October 1976, pages 774—80 S. AKITA et al.:
"Solution Properties of Poly(D-beta-hydroxy-
butyrate). I. Biosynthesis and Characterization"**

(73) Proprietor: **IMPERIAL CHEMICAL INDUSTRIES
PLC**
**Imperial Chemical House Millbank**
**London SW1P 3JF (GB)**

(72) Inventor: **Holmes, Paul Arthur**
**27 Alprahan Crescent**
**Upton by Chester Cheshire (GB)**
Inventor: **Wright, Leonard Frederick**
**8, Blairmore Gardens Eaglescliffe**
**Stockton-on-Tees Cleveland (GB)**
Inventor: **Alderson, Barry**
**5 Sinderby Close**
**Billingham Cleveland (GB)**
Inventor: **Senior, Peter James**
**Foulis Cottage Ingleby Greenhow**
**Middlesbrough Cleveland (GB)**

(74) Representative: Gratwick, Christopher et al,
**Imperial Chemical Industries PLC Legal
Department: Patents Thames House North
Millbank**
**London SW1P 4QG (GB)**

Courier Press, Leamington Spa, England.

# A process for the extraction of poly-3-hydroxybutyric acid from microbial cells

This invention relates to an extraction process and in particular to a process of extracting poly - $\beta$ - hydroxybutyric acid, hereinafter referred to as PHB from microbial cells.

It has been known since the 1920's that many micro-organisms are capable of accumulating granules of PHB within their cells as an energy reserve material. It has been proposed in US—A—3107172 to dry such PHB containing bacterial cells, for example by spray drying, and to use the resultant dried cells as moulding compositions. Suggestions have also been made to extract PHB from bacterial cells and to use it as a plastics material, but methods so far disclosed have not been economically acceptable.

In order to extract the PHB, it is generally necessary to contact the bacterial cells with a solvent in which PHB is soluble to leach out the PHB from the remainder of the bacterial cell material. Some bacteria, for example members of the genus *Azotobacter* readily yield up their PHB to the extraction solvent, whereas other bacteria, eg *Pseudomonadaceae* have more robust cells and require a cell disruption step prior to contact with the extraction solvent.

Methods of extraction previously proposed have included the steps of harvesting the bacterial cells from the aqueous fermentation medium, eg by centrifugation, to give a mass of wet cells which are then contacted with acetone to effect drying and cell breakage. After removal of the acetone, the PHB is extracted with a suitable solvent, eg pyridine (US—A—3036959) or a dichloromethane/ethanol mixture (US—A—3044942). Such methods have the advantage that in addition to effecting drying and cell breakage, acetone also extracts lipids and pigments (if any) which would otherwise contaminate the product. However treatment of a mass of wet cells with acetone to effect drying and cell breakage is not economic on a large scale.

Another method is described in US—A—3275610 wherein a dispersion of bacterial cells in water is subjected to ultrasonic vibration to rupture the cells followed by centrifugation and drying before extraction with a solvent such as chloroform. After separation of the PHB from the chloroform solution, the PHB is washed to extract lipids therefrom.

It has also been proposed in US—A—4101533 to extract PHB from dried cells, or directly from a wet mass of cells harvested from the fermentation medium by centrifugation, by heating the cells with certain cyclic carbonate solvents.

It is also possible to extract PHB directly from the aqueous cell suspension produced by fermentation, preferably after some concentration, by contact with certain solvents such as chloroform, dichloromethane, or 1,2-dichloroethane with, where necessary, a cell disruption step, eg milling, prior to contact with the solvent. However the solvent, and extraction conditions, have to be selected with care to avoid undue uptake by the solvent of non-PHB material particularly lipids and pigment (if any) present in the bacterial cell. Not only does such non-PHB material contaminate the product and so present purification difficulties but also the co-extraction of lipids may tend to result in the formation of a relatively stable emulsion between the solvent and aqueous phases rendering separation thereof difficult. With such a direct extraction process, a separate lipid extraction step prior to contact with the PHB extraction solvent is generally not practical as the solvents that extract lipids would need to be removed, together with the lipids, prior to contact with the PHB extraction solvent and, because the more effective lipid solvents tend to be water miscible, such removal of the lipid solution presents practical difficulties.

We have now found that PHB can be extracted from bacterial cells by a particularly simple process amenable to large scale operation.

According to the present invention we provide a process for the extraction of PHB from an aqueous suspension of PHB-containing bacterial cells comprising introducing said suspension in finely divided form into a current of gas heated to a temperature of at least 100°C to evaporate the water from said suspension, collecting the resultant dried bacterial cells, extracting the PHB therefrom by contact with an extraction solvent which is a liquid that is a solvent from the PHB in the bacterial cells, and separating the extraction solvent having the PHB dissolved therein from the bacterial cell residue.

We have found that such a drying process sufficiently weakens the bacterial cells to enable the PHB to be extracted without the necessity for any separate cell breakage step. While for particularly robust bacteria a separate cell breakage step, eg milling, prior to drying may be desirable to increase the yield of PHB extracted, we have found that generally such a separate cell breakage step prior to drying is not necessary and indeed is best avoided if possible as drying milled dispersions gives rise to difficulties such as build-up in the drier.

In the process of the invention it is preferred to subject the cells to a lipid extraction step prior to extraction with the PHB extraction solvent. Thus the dried cells may be extracted with a lipid/pigment solvent such as acetone, methanol, ethanol, butanol, hexane, or petroleum ether, followed by separation of the solvent containing dissolved lipids/pigment from the cells prior to contacting the cells with the PHB extraction solvent. The lipid/pigment extraction is preferably performed by refluxing the dried cells with the solvent. Acetone and methanol are the preferred lipid extraction solvents. The lipid/pigment extraction solvent may be used in admixture with another PHB non-solvent such as diethyl ether.

It will be appreciated that this lipid extraction may also give rise to some further weakening or breakage of the cells thus facilitating the subsequent extraction of the PHB.

In the process of the invention the cells are separated from the aqueous suspension by a drying process involving introducing the cell suspension in finely divided form, e.g. as a spray or fine stream, into a current of gas, e.g. air, heated to a temperature of at least 100°C. Preferably the suspension is introduced via a spray or atomizing nozzle. Such drying processes are well known and include spray and flash drying.

The heated gas current evaporates off the water which is carried away by the gas stream leaving the dried cells which are collected for extraction with the PHB extraction solvent.

The gas inlet temperature may be in the range 100°C to 500°C and is preferably in the range 120°C to 250°C.

Suitable PHB extraction solvents include pyridine, cyclic carbonates and, particularly, partially halogenated hydrocarbons such as chloroform, dichloromethane and 1,2-dichloroethane. 1,2-Dichloroethane is not normally considered to be a solvent for PHB because PHB, after separation from bacterial cells, does not readily, or completely, dissolve in 1,2-dichloroethane. Thus, whereas the PHB solution separated from the bacterial cell residue appears to be a single phase, a solution made by re-dissolving PHB in 1,2-dichloroethane after precipitation is, except when very dilute, pearly in appearance, and PHB that has been precipitated and dried does not readily re-dissolve in that solvent. It is therefore surprising that such a solvent should be effective for extracting PHB from cells.

For efficient extraction, the extraction is preferably conducted at a temperature above 40°C. Thus temperatures up to, and including, the solvent boiling point may be used and super-atmospheric pressures may be employed to enable temperatures in excess of the solvent boiling point at atmospheric pressure to be employed.

Where the cell suspension is subjected to a cell disruption step, e.g. milling, as is not preferred for reasons described hereinbefore, prior to drying, the extraction temperature should be below 40°C to avoid undue uptake of lipids. Thus if a milled dispersion is dried and extracted with a hot solvent, on precipitation of the PHB from the solvent, a gelatinous sticky mass tends to be formed. Where however a lipid extraction step is employed prior to contact with the PHB solvent, the extraction with the PHB solvent may be conducted at temperatures above 40°C.

The weight of PHB extraction solvent used is preferably 10 to 100 times the cell dry weight.

The use of smaller amounts of solvent may reduce the extraction efficiency of crude PHB and may give solutions of excessive viscosity while the use of larger amounts is uneconomic. The amount of solvent is preferably such that the extracted solution contains 0.5 to 5%, particularly 1 to 2% PHB by weight.

The contacting time for extraction should be a compromise to give adequate extraction without being uneconomically lengthy.

Separation of the cell residue from the PHB-containing solution may be effected by a simple filtration or centrifugation step. If the cells are subjected to a lipid extraction prior to extraction of the PHB, the filtration of the PHB solution from the cell residue tends to be particularly facile and can be effected using relatively coarse filters.

After separation of the PHB-containing extraction solvent from the bacterial cell residue, the PHB solution may be further filtered, if desired, to remove any suspended bacterial fragments. Such filtration is preferably conducted using a filter, e.g. a glass fibre filter, having a pore size of less than 5 $\mu$m, preferably less than 2 $\mu$m.

The separated PHB-containing solution can be used directly, preferably after filtration, for making solvent cast articles such as coatings, films or fibres or the solution may be treated further to separate solid PHB, for example by evaporation of the solvent or by precipitation by addition of the PHB-containing solution to a liquid in which PHB is insoluble and with which the solvent is miscible. Examples of suitable liquids include petroleum ether and methanol/water mixtures. The PHB may be purified, if desired, by washing with methanol or acetone.

After extraction of the PHB, the bacterial cell residues may be further refined for other uses, e.g. as a feedstuff or fertilizer.

Any bacteria that are capable of accumulating PHB may be used to produce the PHB-containing bacterial cells. A paper by Senior et al in Advances in Microbial Physiology 1973 *10* 203—266 lists the bacteria published up to June 1972 and others are described in US Patent 3072538 (*Rhizobium* mutants) and UK Patent 1535632 (especially mutants) of *Alcaligenes eutrophus, Bacillus megaterium, Zoogloea ramigera,* and *Mycoplana rubra*). Among the preferred bacteria are *Azotobacter*, especially *chroococcum, Alcaligenes,* especially *eutrophus,* and the *Pseudomonadaceae,* especially *Pseudomonas* AM1 and *Methylobacterium organophilum.*

Among such bacteria are those capable of metabolising one or more of a variety of substrates, for example carbohydrates, ethanol, methanol, polyhydric alcohols, carbon dioxide/hydrogen, and carboxylic acids, and, according to the substrate used, may grow aerobically or anaerobically. The invention is of particular utility in separating PHB from bacterial cells of the *Pseudomonadaceae* grown under aerobic fermentation conditions on an alcohol, particularly methanol, substrate. The invention is also of particular utility in separating PHB from *Azotobacter* grown on a water soluble carbohydrate such as sucrose or glucose.

The cell suspension produced by the fermentation process will typically contain 20 to 55 g l⁻¹ biomass solids.

The invention is illustrated by the following examples in which all percentages are by weight.

Example 1 (Comparative)

This example demonstrates that simple air drying does not weaken the cells sufficiently to allow efficient PHB extraction.

1000 ml of an aqueous suspension of *Methylobacterium organophilum* (NCIB 11483) containing 60 g biomass solids of which 36% was PHB was centrifuged to give a pellet of wet bacterial cells.

(NCIB No. refers to the number of the culture deposited at the National Collection of Industrial Bacteria, Torry Research Station, Aberdeen, Scotland).

The pellet was then dried in a fluid bed drier at 40°C for 10 hours.

10 g of the resultant dried cells were suspended in 500 ml of 1,2-dichloroethane at room temperature for 15 minutes and then the solvent phase was removed by centrifugation and decanting. This solution was added to 3000 ml of a methanol/water mixture (4 volumes of methanol to 1 volume of water) with vigorous stirring to precipitate crude PHB. The precipitate was collected on a filter and dried in vacuo at 50°C. The yield of crude PHB was less than 0.5%.

The above experiment was repeated but the dry cells were sheared with 1,2-dichloroethane in a Silverson mixer at room temperature for 15 minutes. The yield of crude PHB was 1.4%.

The above experiment was repeated but the dry cells were refluxed with the 1,2-dichloroethane at 83°C for 15 minutes. The yield of crude PHB (purity 94.5%) was 29%.

$$\text{(Yields are calculated herein as } \frac{\text{weight of crude PHB recovered}}{\text{dry weight of cells used}} \times \frac{100}{\text{\% PHB in cells}} \times 100\text{)}$$

Example 2

5000 ml of the aqueous suspension of bacterial cells used in Example 1 was spray dried at a suspension feed rate of 5000 ml hr⁻¹, an air inlet temperature of 150°C, an air outlet temperature of 80°C and an air flow rate of 300 m³ hr⁻¹.

20 g of the spray dried cells were suspended in 1000 ml of 1,2-dichloroethane at room temperature for 15 minutes. The cell debris was removed by filtration through a Whatman 541 paper filter. The PHB was recovered from the solution by adding the latter to 5000 ml of a methanol/water mixture (4 vol. methanol: 1 vol. water) with vigorous stirring. The precipitate was collected on a filter. The yield of crude PHB was 5.7%.

The above experiment was repeated except that the dried cells were sheared with the 1,2-dichloroethane in a Silverson mixer at room temperature for 15 minutes. The yield of crude PHB (purity 98.2%) was 12.2%.

The above experiment was repeated except that the dried cells were refluxed with the 1,2-dichloroethane at 83°C for 15 minutes. The yield of crude PHB (purity 93.6%) was 98.3%. The precipitate was washed 5 times with 500 ml aliquots of methanol and then dried at 106°C. The purity of the washed PHB was 98.7%.

Example 3

20 g of the spray dried cells as used in Example 2 were refluxed for 5 minutes with 60 ml of acetone at 56°C to extract lipids and pigment and then the acetone removed by filtration. The residual cells were then sheared for 15 minutes at room temperature with 1000 ml of 1,2-dichloroethane in a Silverson mixer. The resultant solution was filtered from the cell residue using a Whatman 541 paper filter and the PHB was then precipitated by adding the solution to 5000 ml of a methanol/water mixture (4 vol. methanol:1 vol. water) with vigorous stirring. The precipitate was collected on a filter.

The yield of crude PHB (purity 96.7%) was 43.2%.

The above procedure was repeated save that instead of shearing the acetone extracted cells with 1,2-dichloroethane, the acetone extracted cells were refluxed for 15 minutes with 1,2-dichloroethane at 83°C.

The yield of crude PHB (purity 98.4%) was 95%.

It is thus seen that while the acetone extraction further weakened the spray dried cells to allow more PHB to be extracted by cold 1,2-dichloroethane, spray drying alone sufficiently weakened the cells to permit efficient extraction of the PHB by boiling 1,2-dichloroethane. The acetone extraction did, however, improve the purity of the extracted PHB.

Example 4

An aqueous suspension of cells of *Azotobacter chroococcum* (NCIB 9125) containing 60 g l⁻¹ biomass solids of which 37.8% was PHB was spray dried, acetone extracted, extracted with 1,2-dichloroethane under reflux, and precipitated using the conditions described in Example 3 above.

The yield of crude PHB (purity 98%) was 89.4%.

Example 5

Example 4 was repeated using methanol instead of acetone as the lipid extraction solvent and dichloromethane instead of 1,2-dichloroethane as the PHB extraction solvent. The yield of PHB (purity 98%) was in excess of 95%.

Similar results were obtained using chloroform as the PHB extraction solvent.

**Claims**

1. A process for the extraction of poly - β - hydroxybutyric acid (PHB) from an aqueous suspension of PHB-containing bacterial cells comprising introducing said suspension in finely divided form into a current of gas heated to a temperature of at least 100°C to evaporate the water from the suspension, collecting the resultant dried bacterial cells, extracting the PHB therefrom by contact with an extraction solvent which is a liquid that is a solvent for the PHB in the bacterial cells and separating the extraction solvent having the PHB dissolved therein from the bacterial cell residue.

2. A process according to claim 1 wherein, prior to contact of the dried cells with the extraction solvent, the dried cells are contacted with a liquid in which the PHB is not soluble but in which lipids and/or pigment (if any) in the bacterial cells is soluble.

3. A process according to claim 2 in which the liquid in which PHB is not soluble in acetone or methanol.

4. A process according to any one of claims 1 to 3 in which the gas is heated to a temperature in the range 120 to 250°C.

5. A process according to any one of claims 1 to 4 in which the extraction solvent is a partially halogenated hydrocarbon.

6. A process according to claim 5 in which the extraction solvent is 1,2-dichloroethane, dichloromethane, or chloroform.

7. A process according to any one of claims 1 to 6 in which the dried cells are contacted with the extraction solvent at a temperature above 40°C.

8. A process according to any one of claims 1 to 7 in which the weight of extraction solvent is 10 to 100 times the cell dry weight.

9. A process according to any one of claims 1 to 8 in which the PHB is separated from the extraction solvent by precipitation into a liquid in which PHB is insoluble and with which the extraction solvent is miscible.

**Revendications**

1. Procédé pour l'extraction d'acide poly - β - hydroxybutyrique (PHB) à partir d'une suspension aqueuse de cellules bactériennes contenant du PHB, consistant à introduire cette suspension à l'état finement divisé dans un courant de gaz chauffé à une température d'au moins 100°C pour provoquer l'évaporation de l'eau à partir de la suspension, à recueillir les cellules bactériennes séchées résultantes, à en extraire le PHB par contact avec un solvant d'extraction qui est un liquide constituant un solvant pour le PHB présent dans les cellules bactériennes, et à séparer le solvant d'extraction dans lequel le PHB est dissous des résidus de cellules bactériennes.

2. Procédé suivant la revendication 1, caractérisé en ce que, avant la mise en contact des cellules séchées avec le solvant d'extraction, les cellules séchées sont mises en contact avec un liquide dans lequel le PHB n'est pas soluble mais dans lequel les lipides et/ou les pigments (éventuellement présents) dans les cellules bactériennes sont solubles.

3. Procédé suivant la revendication 2, caractérisé en ce que le liquide dans lequel le PHB n'est pas soluble est de l'acétone ou du méthanol.

4. Procédé suivant l'une quelconque des revendications 1 à 3, caractérisé en ce que le gaz est chauffé à une température comprise entre 120 et 250°C.

5. Procédé suivant l'une quelconque des revendications 1 à 4, caractérisé en ce que le solvant d'extraction est un hydrocarbure partiellement halogéné.

6. Procédé suivant la revendication 5, caractérisé en ce que le solvant d'extraction est du 1,2-dichloréthane, du dichlorométhane ou du chloroforme.

7. Procédé suivant l'une quelconque des revendications 1 à 6, caractérisé en ce que les cellules séchées sont mises en contact avec le solvant d'extraction à une température supérieure à 40°C.

8. Procédé suivant l'une quelconque des revendications 1 à 7, caractérisé en ce que le poids du solvant d'extraction représente de 10 à 100 fois le poids à sec des cellules.

9. Procédé suivant l'une quelconque des revendications 1 à 8, caractérisé en ce qu'on sépare le PHB du solvant d'extraction par précipitation dans un liquide dans lequel le PHB est insoluble et avec lequel le solvant d'extraction est miscible.

**Patentansprüche**

1. Verfahren zur Extraktion von Poly - β - hydroxybuttersäure (PHB) aus einer wäßrigen Suspension von PHB enthaltenden Bakterienzellen, bei dem die Suspension in fein verteilter Form in einen Strom eines auf eine Temperatur von mindestens 100°C erhitzten Gases eingeleitet wird, um das Wasser aus der Suspension verdampfen zu lassen, die erhaltenen, getrockneten Bakterienzellen gesammelt werden, die PHB daraus durch Inberührungbringen mit einem Extraktionslösungsmittel, bei dem es sich um eine Flüssigkeit handelt, die ein Lösungsmittel für die in den Bakterienzellen enthaltene PHB ist, extrahiert wird und das Extraktionslösungsmittel, worin die PHB gelöst ist, von dem Bakterienzellenrückstand abgetrennt wird.

2. Verfahren nach Anspruch 1, bei dem die getrockneten Zellen vor dem Inberührungbringen der getrockneten Zellen mit dem Extraktionslösungsmittel mit einer Flüssigkeit, in der die PHB unlöslich ist, in der jedoch Lipide und/oder Pigment, die ggf. in den Bakterienzellen enthalten sind, löslich sind, in Berührung gebracht werden.

3. Verfahren nach Anspruch 2, bei dem die Flüssigkeit, in der PHB unlöslich ist, Aceton oder Methanol ist.

4. Verfahren nach einem der Ansprüche 1 bis 3, bei dem das Gas auf eine Temperatur im Bereich von 120 bis 250°C erhitzt wird.

5. Verfahren nach einem der Ansprüche 1 bis 4, bei dem das Extraktionslösungsmittel ein teilweise halogenierter Kohlenwasserstoff ist.

6. Verfahren nach Anspruch 5, bei dem das Extraktionslösungsmittel 1,2-Dichlorethan, Dichlormethan oder Chloroform ist.

7. Verfahren nach einem der Ansprüche 1 bis 6, bei dem die getrockneten Zellen mit dem Extraktionslösungsmittel bei einer oberhalb von 40°C liegenden Temperatur in Berührung gebracht werden.

8. Verfahren nach einem der Ansprüche 1 bis 7, bei dem das Gewicht des Extraktionslösungsmittels das 10- bis 100-fache des Trockengewichts der Zellen beträgt.

9. Verfahren nach einem der Ansprüche 1 bis 8, bei dem die PHB durch Ausfällung in eine Flüssigkeit, in der PHB unlöslich ist und mit der das Extraktionslösungsmittel mischbar ist, von dem Extraktionslösungsmittel abgetrennt wird.